# EUROPEAN PATENT SPECIFICATION

(11) **EP 2 849 788 B1**
(45) Date of publication and mention of the grant of the patent: **03.01.2018**
(21) Application number: 13791248.1
(22) Date of filing: 17.05.2013
(51) Int. Cl.: A61K 39/395, A61K 31/4745, A61K 31/7088, A61K 38/17, A61P 29/00

(54) **PROPROTEIN CONVERTASE SUBTILISIN KEXIN 9 (PCSK9) INHIBITORS FOR USE IN TREATING SEPSIS AND SEPTIC SHOCK**
PROPROTEIN-KONVERTASE-SUBTILISIN-KEXIN-9 (PCSK9)-INHIBITOREN ZUR BEHANDLUNG VON SEPSIS UND SEPTISCHER SCHOCK
INHIBITEURS DE LA PROPROTÉINE CONVERTASE SUBTILISINE/KEXINE 9 (PCSK9) POUR LE TRAITEMENT DU SEPSIS ET DU CHOC SEPTIQUE

(30) Priority: 17.05.2012 US 201261648319 P
(43) Date of publication of application: 25.03.2015
(73) Proprietor: Cyon Therapeutics Inc., Vancouver, British Columbia V7Y 1K8 (CA)
(72) Inventor: WALLEY, Keith, R., North Vancouver British Columbia V7N 4M9 (CA); BOYD, John, H, Vancouver British Columbia V6K 2A8 (CA); RUSSELL, James, A., Vancouver British Columbia V6M 1H8 (CA)
(74) Representative: Novitas Patent AB
(86) International application number: PCT/CA2013/000488
(87) International publication number: WO 2013/170367

(56) References cited:
- CN-A- 101 829 113
- HAO-QING ZHANG ET AL: "BERBERINE INHIBITS CYTOSOLIC PHOSPHOLIPASE A2 AND PROTECTS AGAINST LPS-INDUCED LUNG INJURY AND LETHALITY INDEPENDENT OF THE [alpha]2-ADRENERGIC RECEPTOR IN MICE", SHOCK, 1 September 2007 (2007-09-01), page 1, XP055212544, ISSN: 1073-2322, DOI: 10.1097/shk.0b013e318157ea14
- FEI LI ET AL: "Neutral sulfate berberine modulates cytokine secretion and increases survival in endotoxemic mice", ACTA PHARMACOLOGICA SINICA, vol. 27, no. 9, 1 September 2006 (2006-09-01), pages 1199-1205, XP055212541, ISSN: 1671-4083, DOI: 10.1111/j.1745-7254.2006.00368.x
- BRAUTBAR ARIEL ET AL: "Pharmacological strategies for lowering LDL cholesterol: statins and beyond", NATURE REVIEWS. CARDIOLOGY,, vol. 8, no. 5, 1 May 2011 (2011-05-01), pages 253-265, XP009161349, ISSN: 1759-5010
- BANASZEWSKA, A. ET AL.: 'Proprotein convertase subtilisin/kexin type 9: a new target molecule for gene therapy.' CELL MOL. BIOL. LETT. vol. 17, no. 2, June 2012, pages 228 - 239, XP002732020
- DUFF, C.J. ET AL.: 'PCSK9: an emerging target for treatment of hypercholesterolemia.' EXPERT OPIN. THER. TARGETS. vol. 15, no. 2, February 2011, pages 157 - 168, XP009146484
- ABIFADEL, M. ET AL. STRATEGIES FOR PROPROTEIN CONVERTASE SUBTILISIN KEXIN 9 MODULATION: A PERSPECTIVE ON RECENT PATENTS. vol. 20, no. 11, November 2010, pages 1547 - 1571, XP009143040
- XIAO, H-B. ET AL.: 'Berberine inhibits dyslipidemia in C57BL/6 mice with lipopolysaccharide induced inflammation.' PHARMACOL. REP. vol. 64, no. 4, July 2012, pages 889 - 895, XP055177638
- THAIN, K.R. ET AL.: 'A low density lipoprotein (ldl) receptor modulator, Pcsk9, is associated with increased mortality in septic shock.' AMERICAN THORACIC SOCIETY INTERNATIONAL CONFERENCE 2012, XP008175605 Retrieved from the Internet: <URL:http://www. atsj ournals. org/doi/abs/10.1164/ajrccm-conference.2012. 185.1- MeetingAbstracts.A5999> [retrieved on 2013-07-23]

## Description

### RELATED APPLICATIONS

This application claims priority to United States provisional patent application 61/648,319 filed 17 May 2012.

### TECHNICAL FIELD

This invention relates to the field of proprotein convertase subtilisin kexin 9 (PCSK9) inhibitors, for use in the amelioration or treatment of an inflammatory response to infection and to treat complications associated therewith. In particular, the invention relates to the treatment of an inflammatory response to infection and complications associated therewith.

### BACKGROUND

Proprotein convertase subtilisin kexin 9 (PCSK9) is a member of the proprotein convertase family of pro-teases that is thought to be involved in the regulation of lipid metabolism. Loss-of-function (LOF) mutations in PCSK9 are associated with reductions in low-density lipoprotein cholesterol (LDL-C). Similarly, gain-of-function (GOF) mutations in PCSK9 are associated with increases in low-density lipoprotein cholesterol (LDL-C). Elevated plasma LDL-C is a risk factor for the development of atherosclerosis and associated ischemic cardiovascular disease (CVD), such as myocardial infarction and stroke. Plasma LDL-C is bound by the LDL receptor (LDLR) and once bound the LDL-C/LDLR complex undergoes endocytosis. The LDL-C undergoes lysosomal degradation, while the LDLR is then recycled back to the plasma membrane where it can bind more LDL. The binding of LDL-C by LDLR, the subsequent LDL-C degradation and recycling of the receptor to the plasma membrane is somewhat continuous. However, PCSK9 promotes the degradation of the LDLR and thereby prevents the receptor from recycling to the membrane²². As a result, PCSK9 is a target for LDL-C lowering therapies. Statins reduce cholesterol by inhibiting the enzyme HMG-CoA reductase. HMG-CoA reductase is important in the production of cholesterol in the liver. Statin treatment has been reported to reduce the incidence of pneumonial¹ and reduce mortality from in-hospital pneumonia². HDL has also been reported to be protective in sepsis³. However, the evidence for the protective effect of HDL is not conclusive⁴. For example, continuation of pre-hospitalization statin therapy did not improve outcomes in patients hospitalized with sepsis⁵ and actively increasing plasma HDL using cholesteryl ester transfer protein (CETP) inhibitors such as torcetrapib appeared to result in excess sepsis deaths⁶. There have been no pivotal randomized controlled trials to date of statins in sepsis. Thus, there appears to be a clinically important interaction between lipid metabolism and inflammatory pathways, although apparently contradictory observations indicate that our understanding is very incomplete.

The mechanisms involved in the interaction between lipid metabolism and the septic inflammatory response are similarly unclear. Triglyceride-rich lipoproteins contribute by binding to lipopolysaccharide (LPS) and lipoteichoic acid (LTA) fragments of Gram-positive and Gram-negative pathogens, respectively, which are then internalized via the LDL receptor and cleared by the liver, thereby potentially reducing activation of macrophages⁷⁻¹⁰. A number of macrophage-expressed receptors that modulate the inflammatory response, including PPAR and LXR, are activated by cholesterol¹¹. HDL is reported to augment human monocyte responses to LPS by suppressing the inhibitory activity of high concentrations of LPS binding protein (LBP), where apolipoprotein A-II appears to be the active component¹². Statins have multiple effects which may cause immune modulation including reduction of C-reactive protein levels¹³, reduction in NF-κB activation¹⁴, and improving endothelial e-NOS responses thus reducing leukocyte adhesion within the microcirculation¹⁵ and leukocyte recruitment to the infected site¹⁶. Statins also inhibit protein isoprenylation, including farnesylation, which abrogates pro-apoptotic effects of sepsis on splenic lymphocytes¹⁷.

LDL receptor knock-out mice are reported to be protected against lethal endotoxemia and severe gram-negative infections¹⁸. However, potentially contradictory observations have been made, whereby LDL receptor-deficient mice have been reported to be more susceptible to sepsis induced by cecal ligation and puncture (CLP) than corresponding genetic background mice¹⁹. In LDL receptor knock-out mice a number of inflammatory mediators were altered. Prior to CLP, LDL receptor knock-out mice had an elevation in serum amyloid A protein, lipopolysaccharide binding protein (LBP), and soluble CD 14 (sCD14). Following CLP IL-1β increased more in LDL receptor knock-out mice than controls. In experimental models of murine sepsis statin treatment²⁰ and treatment with an apolipoprotein A-1 mimetic protein²¹ appear to be beneficial.

In summary, lipid metabolism pathways have been shown to interact with the inflammatory response, potentially with important clinical consequences. However, the exact mechanisms and whether this effect impacts patient outcome are uncertain.

Proprotein Convertase Subtilisin Kexin 9 (PCSK9) inhibitors have been primarily suggested for the treatment of hypercholesterolemia and associated atherosclerosis. However, PCSK9 inhibitors/silencers have also been suggested for use in cancer metastasis treatments.

### SUMMARY

The present application is based in part on the discovery that a reduction of activity of proprotein convertase subtilisin kexin 9 (PCSK9) reduces the inflammatory response and improves physiologic outcome in mice and in human subjects having an inflammatory response to infection. In particular, the patients tested had one or more of the following inflammatory responses to infection: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, bowel infection, opportunistic infections, HIV/AIDS, endocarditis, bronchiectasis, chronic bronchitis, meningitis, septic arthritis, urinary tract infection, pyelonephritis, necrotizing fasciitis, Group A streptococcus infection, enterococcus infection, Gram positive sepsis, Gram negative sepsis, culture negative sepsis, fungal sepsis, meningococcemia, epiglotittis, E. coli 0157:H7 infection, gas gangrene, toxic shock syndrome, mycobacterial tuberculosis, Pneumocystic carinii infection, pelvic inflammatory disease, Legionella infection, Influenza A infection, Epstein-Barr virus infection, or encephalitis. Furthermore, it was also found that inhibition of PCSK9 resulted in reduced renal failure, renal dysfunction, respiratory failure, respiratory dysfunction, or acute lung injury in human subjects. In particular, inhibition of PCSK9 may result in reduced renal failure, renal dysfunction, respiratory failure, respiratory dysfunction, or acute lung injury in human subjects who have sepsis and septic shock.

In accordance with a first aspect there is provided a method of treating an inflammatory response to infection, the method including: administering a proprotein convertase subtilisin kexin 9 (PCSK9) inhibitor to a subject in need thereof.

In accordance with another aspect there is provided a pharmaceutical composition for treating an inflammatory response to infection, including a PCSK9 inhibitor and a pharmaceutically acceptable carrier.

In accordance with another aspect there is provided a PCSK9 inhibitor for treating an inflammatory response to infection.

In accordance with another aspect there is provided a use of a PCSK9 inhibitor for treating an inflammatory response to infection.

In accordance with another aspect there is provided a use of a pharmaceutical composition comprising a PCSK9 inhibitor and a pharmaceutically acceptable carrier for treating an inflammatory response to infection.

In accordance with another aspect there is provided a use of a PCSK9 inhibitor in the manufacture of a medicament for treating an inflammatory response to infection.

In accordance with another aspect there is provided a commercial package including (a) a PCSK9 inhibitor; and (b) instructions for the use thereof for treating and an inflammatory response to infection.

In accordance with another aspect there is provided a commercial package including (a) a pharmaceutical composition comprising a PCSK9 inhibitor and a pharmaceutically acceptable carrier; and (b) instructions for the use thereof for treating an inflammatory response to infection.

The PCSK9 inhibitor may be an antibody or antigen-binding fragment thereof. The PCSK9 inhibitor may be a monoclonal antibody or antigen-binding fragment thereof. The PCSK9 inhibitor may be: AMG145; 1D05-IgG2; SAR236553/REGN727 (Alirocumab); RN-316; LGT209; or RG7652. The PCSK9 inhibitor may be a peptide mimetic. The PCSK9 inhibitor may be an EGFA domain mimic, EGF-A peptide, a fibronectin based scaffold domain proteins, or a neutralizing PCSK9 variant. The PCSK9 inhibitor may be an antisense oligonucleotide. The PCSK9 inhibitor may be BMS-PCSK9Rx. The PCSK9 inhibitor may be an RNAi molecule. The PCSK9 inhibitor may be LNA ASO or ALN-PCS. The PCSK9 inhibitor may be any PCSK9 inhibitor known to a person of skill in the art.

The subject may be a human. The inflammatory response to infection, may be one or more of: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, bowel infection, opportunistic infections, HIV/AIDS, endocarditis, bronchiectasis, chronic bronchitis, meningitis, septic arthritis, urinary tract infection, pyelonephritis, necrotizing fasciitis, Group A streptococcus infection, enterococcus infection, Gram positive sepsis, Gram negative sepsis, culture negative sepsis, fungal sepsis, meningococcemia, epiglotittis, E. coli 0157:H7 infection, gas gangrene, toxic shock syndrome, mycobacterial tuberculosis, Pneumocystic carinii infection, pelvic inflammatory disease, Legionella infection, Influenza A infection, Epstein-Barr virus infection, or encephalitis. The subject may have septic shock. The subject may have sepsis.

In accordance with another aspect there is provided a method of treating or preventing renal failure, renal dysfunction, respiratory failure, respiratory dysfunction, or acute lung injury, the method including: administering a proprotein convertase subtilisin kexin 9 (PCSK9) inhibitor to a subject in need thereof.

The subject may have an inflammatory response to infection. The PCSK9 inhibitor may be selected from one or more of the following: an antibody or antigen-binding fragment thereof; a peptide mimetic; an antisense oligonucleotide; an RNAi molecule. The PCSK9 inhibitor may be a monoclonal antibody or antigen-binding fragment thereof. The PCSK9 inhibitor may be: AMG145; 1D05-IgG2; SAR236553/REGN727 (Alirocumab); RN-316; LGT209; or RG7652. The PCSK9 inhibitor may be an EGFA domain mimic, EGF-A peptide, a fibronectin based scaffold domain proteins, or a neutralizing PCSK9 variant. The PCSK9 inhibitor may be BMS-PCSK9Rx. The PCSK9 inhibitor may be LNA ASO or ALN-PCS. The subject may be a human. The inflammatory response to infection, may be one or more of: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, bowel infection, opportunistic infections, HIV/AIDS, endocarditis, bronchiectasis, chronic bronchitis, meningitis, septic arthritis, urinary tract infection, pyelonephritis, necrotizing fasciitis, Group A streptococcus infection, enterococcus infection, Gram positive sepsis, Gram negative sepsis, culture negative sepsis, fungal sepsis, meningococcemia, epiglotittis, E. coli 0157:H7 infection, gas gangrene, toxic shock syndrome, mycobacterial tuberculosis, Pneumocystic carinii infection, pelvic inflammatory disease, Legionella infection, Influenza A infection, Epstein-Barr virus infection, or encephalitis.

Furthermore, a person of skill in the art would appreciate that a subject may also be evaluated to determine whether they have one or more PCSK9 GOF or LOF alleles, as described herein, that may influence the urgency with which a subject is administered a PCSK9 inhibitor. For example, a subject having one or more GOF PCSK9 allele(s) may be considered for earlier and more aggressive management than a subject having a PCSK9 LOF allele. For example, a subject having an rs644000 G allele (LOF) would be less at risk of an unfavourable outcome as compared to a subject having an rs644000 A allele (GOF). Accordingly, a subject having an rs644000 A allele (GOF) or any other PCSK9 GOF allele may be treated with a PCSK9 inhibitor sooner than a subject having an rs644000 G allele (LOF) or any other PCSK9 LOF allele. Nevertheless, the results provided herein demonstrate that both subjects having a GOF and a LOF PCSK9 allele could benefit from PCSK9 inhibition if the subject has an inflammatory response to infection. The invention is defined in the claims.

### BRIEF DESCRIPTION OF THE DRAWINGS

In drawings which illustrate embodiments of the invention:

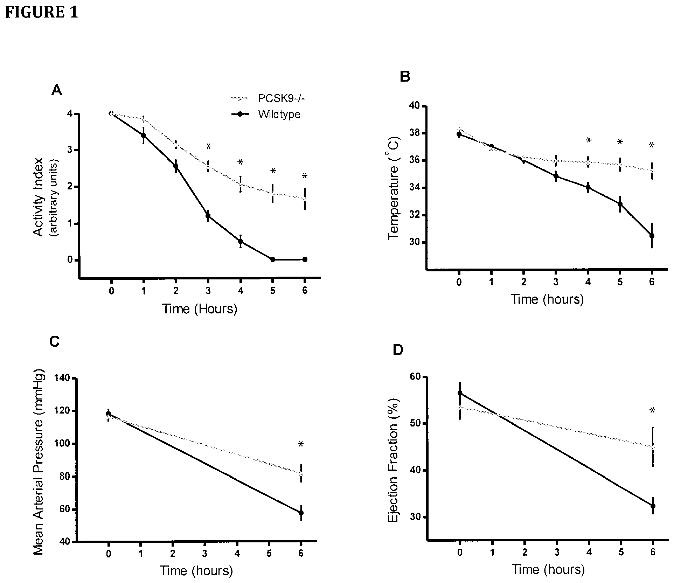

**FIGURE 1** shows a comparison of the (A) activity index, (B) body temperature (°C), (C) mean arterial pressure (mmHg), and ejection fraction (%) phenotypes between background strain mice (C57BL/6 - wild-type) and PCSK9 knock-out mice (PCSK9-/-), following LPS administration;

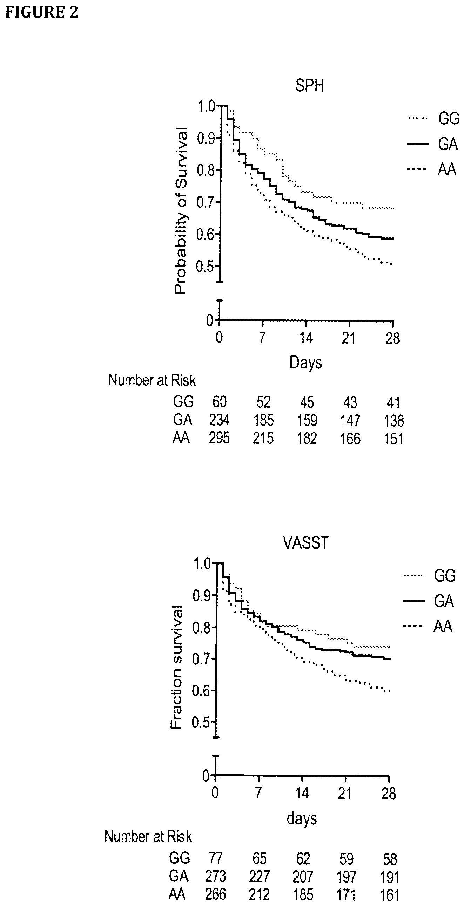

**FIGURE 2** shows survival curves for SPH and VASST patients by rs644000 genotype;

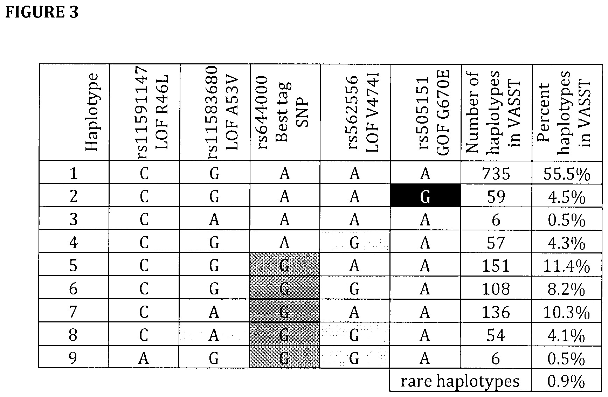

**FIGURE 3** shows a table of haplotypes as resolved in VASST using PHASE²⁸ and haplotypes with MAP≥0.5% are displayed, were the minor allele is shaded for each SNP, where out of the 1324 total observed haplotypes, a LOF allele was observed 442 times within 309 haplotypes and 83.5% of these LOF alleles were contained within haplotypes that also contained the rs644000 minor allele (Haplotypes 6-9) and only 15.5% of the LOF alleles were contained within rs644000 major allele haplotypes (Haplotypes 3 and 4), which shows that the minor allele is a marker of the most common PCSK9 LOF genetic variants (haplotypes 8 and 9 contain 2 LOF alleles, in contrast to the PCSK9 GOF variant was observed within a haplotype that contained the rs644000 major allele (Haplotype 2));

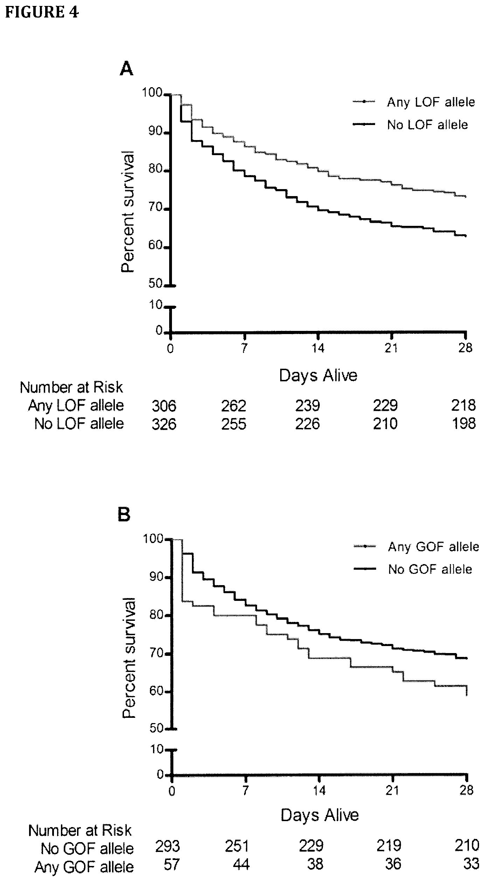

**FIGURE 4A** shows a LOF KM survival curves for VASST patients, wherein having at least one PCSK9 LOF allele had decreased mortality over 28 days (LOFall = 1, top line, n total = 306 with 89 deaths, 29.1% 28-day mortality) compared to patients without a LOF allele (bottom line, n total = 326 with 129 deaths, 39.6% 28-day mortality) (p=0.0037 by log-rank test);
**FIGURE 4B** shows a GOF KM survival curves in VASST patients, wherein having at least one PCSK9 GOF allele (GOFall = 1, bottom line, n total = 57 with 25 deaths, 43.9% mortality at 28 days) had increased mortality over 28 days compared to patients having at least one PCSK9 LOF allele but no GOF alleles (top line, n total = 293 with 83 deaths, 28.3% mortality at 28 days) (p=0.011 by log-rank test); and

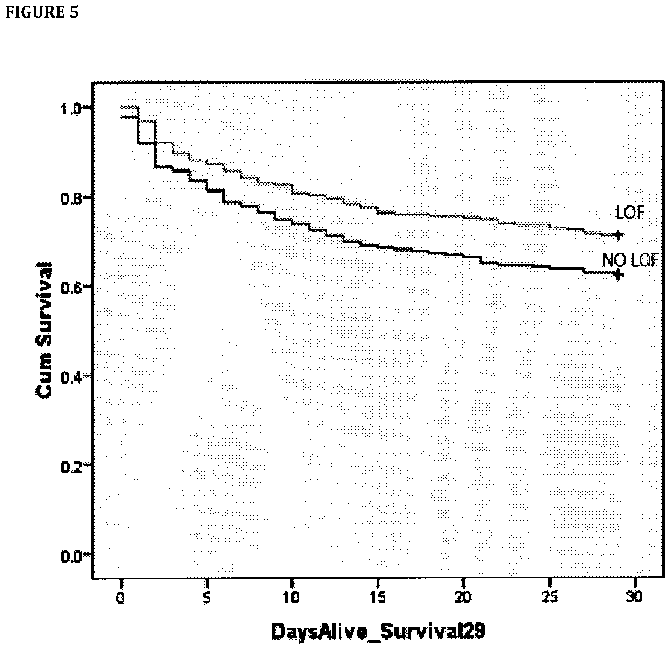

**FIGURE 5** shows LOF KM survival curves in VASST patients who were heterozygous for rs644000 to address the issue of whether the minor allele of rs644000 (or another SNP in high linkage disequilibrium) was the likely function SNP leading directly to improved outcome or, alternatively, whether rs644000 simply marked a preponderance of LOF alleles, the effect of LOF alleles within rs644000 heterozygotes (n=478) was tested, and the rs644000 heterozygous patients in VASST having at least one PCSK9 LOF allele (LOF = 1, top line, n total = 253 with 73 deaths, 71.1% survival at 28 days) had decreased mortality over 28 days compared to patients without a LOF allele (bottom line, n total = 225 with 85 deaths, 62.2% survival at 28 days) (p=0.029 by log-rank test).

### DETAILED DESCRIPTION

Various alternative embodiments and examples are described herein. These embodiments and examples are illustrative and should not be construed as limiting the scope of the invention.

In view of the interaction of lipid metabolism and inflammatory pathways, the present application examined whether PCSK9 alters the systemic inflammatory response to LPS injection in mice. It was found that PCSK9 knock-out mice had a diminished inflammatory response to LPS and were protected against adverse aspects of the physiologic phenotype of a severe inflammatory response, compared to background control mice. To determine whether this observation could have clinically significant consequences the inventors examined genetic polymorphisms of PCSK9 in human sepsis. PCSK9 genetic variants have been well characterized, so it was possible to parse and sort several common variants into known loss-of-function (LOF) variants and compare these to a known gain-of-function (GOF) variant. PCSK9 variants were genotyped in two cohorts of patients with severe sepsis and septic shock. In accord with the murine LPS results, humans with septic shock carrying LOF variants of PCSK9 had a reduced inflammatory cytokine response and decreased mortality whereas GOF variants had the opposite effect.

As used herein, "proprotein convertase subtilisin kexin 9" or "PCSK9" is meant to refer to an important protein in LDL cholesterol (LDL-C) metabolism. PCSK9 plays an important role in the degradation of the LDL receptor (LDLR). In LDL metabolism the LDLR binds to LDL in circulating blood and internalizes the LDL into clathrin-coated pits for lysosomal degradation. Following internalization of the LDL, the LDLR is then recycled back to the plasma membrane where it can bind more LDL. This process repeats continuously. However, PCSK9 degradation of the LDLR prevents recycling of the LDLR to the membrane and thus reduces LDL clearance from the blood. Accordingly, PCSK9 has an important target for inhibition for the promotion of reduced LDL-C and thus a therapeutic target for the treatment of hypercholesterolemia and associated cardiovascular diseases. The crystal structure of PCSK9 was described in PCT/US2008/056316. Furthermore, PCT/IB2004/001686 describes mutations in the human PCSK9 gene associated with hypercholesterolemia. PCSK9 is a part of the LDL-C metabolism pathway.

The PCSK9 gene encodes a pro-protein, which has also been termed Narc 1, a proteinase that is related to proteinase K³⁸. PCSK9 is synthesized as a 74 kDa pro-protein that undergoes cleavage in the endoplasmic reticulum resulting in secretion of a ∼14 kDa fragment and a ∼60 kDa fragment held together by non-covalent bonds^{39,40}. Further autocatalytic cleavage of the ∼14 kDa fragment renders the pro-protein active. The active PCSK9 protein circulating in the plasma binds the LDL receptor and, after internalization, prevents recycling of the receptor back to the cell surface and promotes degradation of the receptor in the lysosome^{41,42}. Inhibition of PCSK9 results in decreased LDL cholesterol levels in humans⁴³.

As used herein, "proprotein convertase subtilisin kexin 9 inhibitor" or "PCSK9 inhibitor" is meant to refer to any molecule that is capable of reducing the normal activity of PCSK9 within a subject upon or after administration of the inhibitor. Such inhibitors may be antibodies (including monoclonal antibodies), other peptides (for example, an EGFA domain mimetic, and EGF-A peptide, a fibronectin based scaffold domain proteins, or a neutralizing PCSK9 variant (for example, with a Pro/Cat domain). Alternatively, the PCSK9 inhibitor may be a nucleic acid molecule (for example, a RNA interference (RNAi), a small interfering RNA (siRNA), a meroduplex RNA (mdRNA), a locked nucleic acid antisense oligonucleotide (LNA) etc.). Furthermore, the PCSK9 inhibitor may be a small molecule inhibitor of PCSK9.

'RNAi' as used herein is meant to include any of the gene silencing methods known in the art, including post-transcriptional gene silencing (PTGS) methods. These may include, but are not limited to any one or more of the following: microRNA (miRNA); small interfering (siRNA); short-hairpin RNA (shRNA); primary-microRNA (pri-miRNA); asymmetric interfering RNA (aiRNA); small internally segmented RNS (sisiRNA); meroduplex RNA (mdRNA); RNA-DNA chimeric duplex; trans-kingdom RNA (tkRNA); tRNA-shRNA; tandem siRNA (tsiRNA); tandem hairpin RNA (thRNA); pri-miRNA mimic cluster; and transcriptional gene silencing (TGS).

As used herein, "monoclonal antibody" or "MAb" is meant to refer to an antibody from a population of substantially homogeneous antibodies (i.e. where the individual antibodies are identical to one another, with the possible exception of some naturally-occurring mutations). MAbs are highly specific, being directed against a single antigenic site and is often directed against a single determinant on an antigen.

As used herein, "humanized" antibody is meant to refer to forms of non-human (e.g., murine) antibodies that are chimeric immunoglobulins, immunoglobulin chains, or fragments thereof (such as Fv, Fab, Fab', F(ab')2 or other antigen-binding subsequences of antibodies) that contain minimal sequence derived from non-human immunoglobulin. Many humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity.

### PCSK9 INHIBITORS

Exemplary PCSK9 inhibitors are described below.

### Monoclonal antibodies

Monoclonal antibodies (MAbs) that specifically bind to PCSK9 are capable of inhibiting PCSK9 activity. In some instances, the MAbs bind near the catalytic domain, which interacts with the low density lipoprotein receptor (LDLR) thereby inhibiting the catalytic activity of PCSK9 on LDLR. A number of these MAbs are in clinical trials (for example, AMG145 (Amgen), 1D05-IgG2 (Merck & Co.), and SAR236553/REGN727/Alirocumab (Aventis/Regeneron)). Similarly, additional MAbs targeting PCSK9 are also in development (for example, RN-316 (Pfizer); LGT209 (Novartis); RG7652 (Roche/Genentech)). A number of PCSK9 inhibitory Antibodies and fragments thereof are described in the patent literature as follows:
**MERCK/SCHERING CORP.** (PCT/US2008/081311);
**SCHERING CORP.** (PCT/US2011/056649);
**REGENERON PHARMACEUTICALS, INC.** (PCT/US2012/054756; PCT/US2012/048574; PCT/US2009/068013);
**SANOFI** (PCT/EP2012/051318; PCT/EP2012/051320; PCT/EP2012/051321);
**ELI LILLY AND COMPANY** (PCT/US2012/054737);
**AFFIRIS AG** (PCT/EP2012/067950);
**PFIZER** (PCT/IB2012/053534; PCT/IB2012/050924; PCT/IB2010/053784);
**NOVARTIS AG** (PCT/EP2012/061045; PCT/US2012/041214; PCT/EP2008/054417);
**IRM LLC and NOVARTIS AG** (PCT/US2012/024633; PCT/US2010/059959);
**GENENTECH INC. and HOFFMANN LA ROCHE** (PCT/US2011/024633);
**MERCK SHARP & DOHME** (PCT/US2010/054714; PCT/US2010/054640; PCT/US2010/048849);
**RINAT NEUROSCIENCE CORP/PFIZER** (PCT/IB2009/053990);
**MERCK & CO INC.** (PCT/US2009/033369; PCT/US2009/033341; PCT/US2007/023223; PCT/US2007/023213; PCT/US2007/023212; PCT/US2007/023169); and
**AMGEN INC.** (PCT/US2008/074097).

PCSK9-mediated activity on cell surface LDLRs has been reversed using antibodies that recognize epitopes on PCSK9. In particular, where those epitopes are associated with the catalytic domain. Intravenous infusion of an Amgen monoclonal antibody (AMG145) specific for the catalytic domain of PCSK9 resulted in a significant reduction of circulating LDL-C levels as early as 8 hours after injection in non-human primates. Merck & Co.'s monoclonal antibody (1D05-IgG2) structurally mimics the EGFA domain of the LDLR. A single injection of 1D05-IgG2 was also found to antagonize PCSK9 function in non-human primates, resulting in reduced plasma LDL-C levels by up to 50%. Pfizer-Rinat and Sanofi-Aventis/Regeneron also have monoclonal antibodies (RN316 and SAR236553/REGN727, respectively), which are also in clinical trials.

### Peptides

Peptides that mimic the EGFA domain of the LDLR that binds to PCSK9 have been developed to inhibit PCSK9. Similarly, EGF-A peptides, fibronectin based scaffold domain proteins, which bind PCSK9, and neutralizing PCSK9 variants (for example, with a Pro/Cat domain), have been developed and all of which have been shown to inhibit PCSK9 activity.

A number of PCSK9 inhibitory peptides are described in the patent literature as follows:
**SCHERING CORP.** (PCT/US2009/044883);
**GENENTECH INC. and HOFFMANN LA ROCHE** (PCT/US2012/043315);
**SQUIBB BRISTOL MYERS CO.** (PCT/US2011/032231; PCT/US2007/015298);
**ANGELETTI P IST RICHERCHE BIO** (PCT/EP2011/054646); and
**AMGEN INC.** (PCT/US2009/034775).

### Oligonucleotides

PCSK9 antisense oligonucleotide from Isis Pharmaceuticals/Bristol-Myers Squibb (BMS-PCSK9Rx) has been shown to increase expression of the LDLR and decrease circulating total cholesterol levels in mice.

Similarly, a locked nucleic acid from Santaris Pharma (LNA ASO) reduced PCSK9 mRNA levels in mice. LNA ASO is complementary to the human and mouse PCSK9 mRNA (accession # NM174936 and NM153565) is a 13-nucleotide long gapmer with the following sequence: GTctgtggaaGCG (uppercase LNA, lowercase DNA) and phos- phorothioate internucleoside linkages.

Alnylam Pharmaceuticals has shown positive results in clinical trials for an siRNA (ALN-PCS) for the inhibition of PCSK9. The siRNA was incorporated into lipidoid nanoparticles to minimize toxicity and intravenously infused in rats, mice, and monkeys, resulting in reduced LDL-C levels after administration.

A number of PCSK9 inhibitory oligonucleotides are described in the patent literature as follows:
**SANTARIS PHARMA A/S** (PCT/EP2007/060703; PCT/EP2009/054499; PCT/EP2010/059257);
**ISIS PHARMACEUTICAL INC.** (PCT/US2007/068404);
**SIRNA THERAPEUTICS INC.** (PCT/US2007/073723);
**ALNYLAM PHARMACEUTICALS INC.** (PCT/US2011/058682; PCT/US2010/047726; PCT/US2010/038707; PCT/US2009/032743; PCT/US2007/068655);
**RXI PHARMACEUTICALS CORP.** (PCT/US2010/000019)
**INTRADIGM CORP.** (PCT/US2009/036550); and
**NASTECH PHARM CO.** (PCT/US2008/055554).

### Small Molecules

Serometrix has reported a small molecule inhibitor of PCSK9 (SX-PCSK9)⁵³. Similarly, berberine as described in the examples may be used as a PCSK9 inhibitor.

### Systemic Inflammation

An "inflammatory response to infection", as used herein, may be selected from one or more of: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, bowel infection, opportunistic infections, HIV/AIDS, endocarditis, bronchiectasis, chronic bronchitis, meningitis, septic arthritis, urinary tract infection, pyelonephritis, necrotizing fasciitis, Group A streptococcus infection, enterococcus infection, Gram positive sepsis, Gram negative sepsis, culture negative sepsis, fungal sepsis, meningococcemia, epiglotittis, E. coli 0157:H7 infection, gas gangrene, toxic shock syndrome, mycobacterial tuberculosis, Pneumocystic carinii infection, pelvic inflammatory disease, Legionella infection, Influenza A infection, Epstein-Barr virus infection, or encephalitis.

The "complications associated with inflammatory response to infection" may be selected from renal failure; renal dysfunction; respiratory failure; respiratory dysfunction; or acute lung injury. A PCSK9 inhibitor may be used for the treatment of or prevention of or amelioration of any complication associated with inflammatory response to infection, for example, renal failure; renal dysfunction; respiratory failure; respiratory dysfunction; or acute lung injury.

As used herein "treatment" is meant to include the treatment of or the prevention of or the amelioration of a disease or condition or symptom.

As used herein "systemic inflammatory response syndrome" or "SIRS" is defined as including both septic (i.e. sepsis or septic shock) and non-septic systemic inflammatory response (i.e. post operative). "SIRS" is further defined according to ACCP (American College of Chest Physicians) guidelines as the presence of two or more of A) temperature >38°C or <36°C, B) heart rate >90 beats per minute, C) respiratory rate >20 breaths per minute, and D) white blood cell count >12,000 mm³ or <4,000 mm³.

"Sepsis" is defined as the presence of at least two "SIRS" criteria and known or suspected source of infection. Septic shock was defined as sepsis plus one new organ failure by Brussels criteria plus need for vasopressor medication.

An "rs" prefix designates a SNP in the database is found at the NCBI SNP database. The "rs" numbers are the NCBI|rsSNP ID form.

### Methods & Materials

### Mice

All animal studies were approved by the University of British Columbia animal ethics committee and conformed to NIH and Guide for the Care and Use of Laboratory Animals guidelines.

Male C57BL/6 (background control strain) and PCSK9 knock-out (B6:129S6-Pcsk9tm1Jdh/J) mice, body weight 25-30 grams, 10-14 weeks old, were obtained from Jackson Labs™. Compared to background controls, PCSK9 knock-out mice had reduced plasma cholesterol concentrations with nearly undetectable LDL cholesterol concentrations²³. There are no other reported obvious phenotypic alterations.

### Lipopolysaccharide induced systemic inflammation

Healthy mice had activity level, body temperature, blood pressure, and cardiac function (echocardiography) assessed at baseline (time 0) as detailed below. Mice were then injected intra-peritoneally with LPS (20 mg/kg, E. coli strain 01 II: B4, Sigma™, St. Louis, MO). This dose was determined from previous experiments where, in the background strain of C57BL/6 mice, LPS 20 mg/kg delivered intra-peritoneally was the lowest dose, which was lethal in all cases within 12 hours²⁴. Then activity level and temperature were measured every hour for six hours. At six hours blood pressure was measured by invasive arterial cannulation and cardiac function (echocardiography) was assessed again.

### Physiologic assessment of mice

Activity Index: Mice were observed during 2 minutes for posture and activity. 0 (normal) denotes that there are no times with a hunched posture, and the mouse had spontaneous rapid movements interspersed with eating and drinking. 1 (mild) is occasional brief (5-20 seconds) hunched posture, which spontaneously reverted to normal with ongoing spontaneous rapid movements interspersed with eating and drinking. 2 (mild-moderate) was longer (>20 seconds) in the hunched posture which spontaneously reverted to normal with ongoing spontaneous rapid movements interspersed with eating and drinking. 3 (moderate-severe) was nearly continual hunched posture with movement only when subjected to strong external stimuli. 4 (severe) was continual hunched posture without movement. 0.5 intervals were used if the mouse exhibited both levels within one observation period.

Temperature: Temperature was measured hourly using an infra-red thermometer (IR-101 La Crosse Technology, La Crosse USA)) held 2-3 mm from the abdomen.

Blood Pressure: At baseline (time 0) mean arterial blood pressure was measured using a non-invasive tail cuff (CODA 2™, Kent Scientific, Torrington, USA). Six hours after LPS injection, mice were anesthetized using inhaled isofluorane (1-3%). Following a small laparatomy, the abdominal aorta was punctured using a 27 gauge needle then a number 2 French micromanometer catheter (Mikro-tip SPR-838™, Millar Instruments Inc., Houston, TX) was inserted into the aorta. Mean arterial pressure was derived using analysis software (PVAN 2.9™, Millar Instruments Inc.).

Echocardiography: Mice were lightly anaesthetized using inhaled isofluorane (1-3%) and placed on a warming blanket. M-mode echocardiograms (ECHO) were targeted from 2D echos obtained using the Vevo 770 ECHO (Visualsonics™, Toronto, ON, Canada) operating at a 120 Hz frame rate. Left parasternal 2D left ventricular cross-sectional echocardiographic images were obtained. The position and angle of the echo transducer is maintained by directing the beam just off the tip of the anterior leaflet of the mitral valve and by maintaining internal anatomic landmarks constant. All measurements are taken from M-mode traces at end-expiration. Left ventricular internal dimensions were measured at end-diastole (defined as the onset of the QRS complex in lead II of the simultaneously obtained electrocardiogram) and at end-systole (defined as minimum internal ventricular dimension).

Multiplex cytokine assay: Plasma was diluted 0-50X to ensure that all measurements were within test range and 50 µL per microplate well was added to duplicate wells to the LUM000 mouse base kit microplate (R&D Systems™, Minneapolis, MN), and the protocol followed as per the manufacturer's instructions. Each sample was measured in duplicate. Microplates were analyzed using the Luminex100™ with accompanying 1.7 Software (Luminex Corporation™, Austin, TX). Cytokines were selected to represent early inflammation (TNFα), an integrated inflammatory marker (IL-6), an anti-inflammatory marker (IL-10), and representative CC chemokines (JE as a murine homologue of human MCP-1) and CXC chemokines (MIP-2 and KC, which are murine homologues of human IL-8).

### Human genetic association study

St Paul's Hospital (SPH) Derivation Cohort. All patients admitted to the ICU at St. Paul's Hospital in Vancouver, Canada between July 2000 and January 2004 were screened and of these, 601 patients were classified as having septic shock and had DNA available. Septic shock was defined by the presence of two or more diagnostic criteria for the systemic inflammatory response syndrome, proven or suspected infection, new dysfunction of at least one organ, and hypotension despite adequate fluid resuscitation²⁵. Twelve patients were excluded as they had been included in the VASST cohort (see below), leaving the remaining 589 patients for further study. Inclusion criteria and clinical phenotyping are described elsewhere²⁶. The Institutional Review Board at St. Paul's Hospital and the University of British Columbia approved the study.

Furthermore, each of the patients evaluated in the study had an inflammatory response to infection. Accordingly, each of the patients would have had one or more of the following: sepsis, septicemia, pneumonia, septic shock, systemic inflammatory response syndrome (SIRS), Acute Respiratory Distress Syndrome (ARDS), acute lung injury, infection, pancreatitis, bacteremia, peritonitis, abdominal abscess, bowel infection, opportunistic infections, HIV/AIDS, endocarditis, bronchiectasis, chronic bronchitis, meningitis, septic arthritis, urinary tract infection, pyelonephritis, necrotizing fasciitis, Group A streptococcus infection, enterococcus infection, Gram positive sepsis, Gram negative sepsis, culture negative sepsis, fungal sepsis, meningococcemia, epiglotittis, E. coli 0157:H7 infection, gas gangrene, toxic shock syndrome, mycobacterial tuberculosis, Pneumocystic carinii infection, pelvic inflammatory disease, Legionella infection, Influenza A infection, Epstein-Barr virus infection, or encephalitis.

Vasopressin and Septic Shock Trial (VASST) Validation Cohort. VASST was a multicenter, randomized, double blind, controlled trial evaluating the efficacy of vasopressin versus norepinephrine in 779 patients who had septic shock, as defined above²⁵, and vasopressor infusion of at least 5 µg/min of norepinephrine, or equivalent²⁷. Inclusion criteria and clinical phenotyping are described elsewhere²⁷. DNA was available from 616 patients. The research ethics boards of all participating institutions approved this trial and written informed consent was obtained from all patients or their authorized representatives. The research ethics board at the coordinating center (The University of British Columbia) approved the genetic analysis.

Genotyping and SNP Selection: DNA was extracted from buffy coat of discarded blood samples using a QIAamp DNA™ maxi kit (Qiagen™, Mississauga, ON, Canada) (SPH cohort) or QIAamp DNA Blood Midi Kit™ (Qiagen™) (VASST cohort). Tag SNP genotyping was performed using the Illumina Golden Gate™ assay (Illumina Inc., San Diego, CA). To select initial tag SNPs in PCSK9 for genotyping we first resolved haplotypes using PHASE²⁸ applied to dense CEU genotyping of PCSK9 available from SeattleSNPs. A crossing over event is evident at approximately the middle of the gene resulting in the requirement for a large number of tag SNPs to resolve all possible haplotypes. To simplify genotyping, we limited our tag SNP choices to three bins within the 3' end of the gene and one further bin 5' to the crossing over event. We weighted our tag SNP choice within each bin by the genotyping probability of success supplied by Illumina for the Golden Gate™ assay. Accordingly, PCSK9 tag SNPs were genotyped in both septic shock cohorts were rs644000, rs2479408, rs2479409 and rs572512. Subsequently, additional known loss-of-function (LOF) SNPs (rs11591147 R46L, rs11583680 A53V, rs562556 V474I) and a known gain-of-function (GOF) SNP (rs505151 G670E) were genotyped in the VASST cohort as part of whole genome genotyping using the Illumina Human 1M-Duo™ genotyping platform (Illumina Inc.).

Primary and Secondary Outcomes: The primary outcome was 28-day mortality. Secondary outcomes included measures of organ dysfunction, which were calculated as days alive and free of organ dysfunction²⁹. We assessed cardiovascular, respiratory, renal, hematologic, hepatic, and neurologic dysfunction as well as need for vasopressors, mechanical ventilation and renal replacement therapy. We also assessed the systemic inflammatory response by measuring plasma cytokine levels in 278 patients from VASST cohort at baseline and at 24 hours after inclusion into VASST.

Cytokine measurements: Human multiplex kits (EMD Millipore™) were used according to the manufacturer's recommendations with modifications as described below. Briefly, samples were mixed with antibody-linked magnetic beads on a 96-well plate and incubated overnight at 4°C with shaking. Plates were washed twice with wash buffer in a Biotek ELx405™ washer. Following a one-hour incubation at room temperature with biotinylated detection antibody, streptavidin-PE was added for 30 minutes with shaking. Plates were washed as above and PBS added to wells for reading using a Luminex 200™ (Illumina Inc.) with a lower bound of 100 beads per sample per cytokine. Each sample was measured in duplicate. To correspond to the mouse cytokine measurements we selected cytokines to represent early inflammation (TNFα), an integrated inflammatory marker (IL-6), an anti-inflammatory marker (IL-10), and a representative CC chemokine (MCP-1) and CXC chemokine (IL-8).

### Statistical Analysis

We used repeated measures analysis of variance to test for differences in activity level, temperature, and ejection fraction between PCSK9 knock-out and background control mice over time. We used unpaired t-tests to test for differences in mean arterial pressure because this variable was measured using two different instruments at baseline and at 6 hours after LPS administration.

For human septic shock our primary analysis used logistic regression to determine the risk of mortality by PCSK9 genotype, including the covariates of age, gender, Caucasian ancestry, and a surgical versus medical primary diagnosis in the statistical model. We tested for differences in cytokine concentrations by genotype using a repeated measures analysis of variance. Univariate analyses were performed using chi-square tests for categorical data and either Kruskal-Wallis tests or one-way ANOVA for continuous data. Populations were tested for Hardy-Weinberg equilibrium using a chi-square test. All tests were two-sided. Differences were considered significant if P<0.05. All analyses were performed using R (version 2.8.1, www.R-project.org) and SPSS™ version 16.0 (SPSS Inc, Chicago, IL) statistical software packages.

### EXAMPLES

### EXAMPLE 1: PCSK9 knock-out mice have a blunted response to LPS

By six hours after LPS injection all 10 C57BL/6 background control mice exhibited continuously hunched posture and did not move despite strong stimulus (Activity index of 4/4). In contrast the PCSK9 knock-out mice had a mean activity index of 2.40 ± 0.90 (p<0.05 vs. C57 mice) corresponding to at most 20-30 seconds of hunched posture which spontaneously reverted to normal with ongoing spontaneous rapid movements interspersed with eating and drinking (FIGURE 1A). A comparison of group means demonstrates a statistically significant effect (* p<0.05) in hours 3-6, with typically an activity index greater than 3.5 represents a terminal state.

Background control type mice (C57BL/6) demonstrated a progressive loss of body temperature over the six hours following LPS administration such that 6 of the 10 mice had a body temperature < 32°C and the group mean temperature at six hours was 30.5 ± 2.8°C (FIGURE 1B), while none of the 10 PCSK9 knock-out mice had their temperature drop below 32°C and the group mean temperature was 35.2 ± 1.8°C (p<0.05 PCSK9 knock-out versus background control). A comparison of group means demonstrates a statistically significant effect (p<0.05) in hours 5 and 6, with typically a sustained temperature less than 32°C represents a terminal state.

LPS induced an acute decrease in mean arterial pressure and left ventricular ejection fraction within hours in C57BL/6 background control mice^{24,30}. As shown in TABLE 1A and FIGURE 1C, in the background control mice the mean arterial pressure declined from 120 ± 5 mmHg to 63 ± 11 mmHg at six hours following LPS administration. PCSK9 knock-out mice had a similar mean arterial pressure at baseline time 0 (110 ± 6 mmHg), but demonstrated far less decline in mean arterial pressure at 6 hours after LPS administration (75 ± 15 mmHg) compared to background control mice (P<0.05) (TABLE 1A). Similar results were obtained when the ejection fraction (%) were compared (see FIGURE 1D).

When compared to the results with LPS treated LDLR-/- mice also treated with either saline or berberine, there were minimal differences between the saline and berberine mice (TABLE 1B). This is consistent with the hypothesis that the benefit derived from the inhibition of PCSK9 is the result of increased availability of LDLR and a subsequent reduction in plasma LDL.

**TABLE 1A**

| Mouse hemodynamic response following 20 mg/kg LPS or sterile saline. n ≥ 6 per group. Data are mean ± standard deviation. | | | | | | |
|---|---|---|---|---|---|---|
| | **HR bpm** (Saline) | **HR bpm** (LPS) | **MAP** mmHg (Saline) | **MAP** mmHg (LPS) | **LVEF %** (Saline) | **LVEF %** (LPS) |
| Background control | 370 ± 38 | 450 ± 53 | 120 ± 5 | 63 ± 11 | 57 ± 6.2 | 32 ± 4.9 |
| PCSK9 knock-out | 510 ± 39* | 590 ± 35* | 110 ± 6 | 75 ± 15* | 54 ± 6.8 | 44 ± 8.8* |

| | | | | | | |
|---|---|---|---|---|---|---|
| • P<0.05 PCSK9 knock-out compared to Background control. | | | | | | |

**TABLE 1B**

| Responses to LPS treatment in LDLR-/- mice also treated with either saline or berberine. | | | | | | | | |
|---|---|---|---|---|---|---|---|---|
| | **Activity Baseline** | **Activity 6 hrs LPS** | **Temp. Baseline** | **Temp. 6 hrs LPS** | **EF Baseline** | **EF 6 hrs LPS** | **MAP Baseline** | **MAP 6 hrs LPS** |
| **Saline** | 0 (0-0) | 0 (-0.8-0.3) | 37.1 (36.2-37.9) | 28.3 26.0-30.6) | 54.2 36.6-71.8) | 21.3 (16.3-26.3) | 124 118-129) | 53 (32-74) |
| **Berberine** | 0 (0-0) | 0 (-0.8-0.3) | 37.0 (36.2-37.9) | 28.5 26.1-30.8) | 50.4 38.2-62.6) | 26.0 (23.0-29.0) | 109 (93-125) | 53 (52-54) |
| **P-value** | NA | 0.356 | 0.806 | 0.898 | 0.844 | 0.045 | 0.565 | 0.477 |

| | | | | | | | | |
|---|---|---|---|---|---|---|---|---|
| • (Data are median (95% CI) and all groups were n=4) | | | | | | | | |

As assessed by trans-thoracic echocardiography left ventricular ejection fraction declined significantly from baseline (57 ± 6.2%) to six hours after LPS administration (32 ± 4.9%, p<0.05) in background control mice. In contrast, in the PCSK9 knock-out mice left ventricular ejection fraction was similar to the background control mice at baseline (54 ± 6.8%), but had far less decline six hours after LPS administration (44 ± 8.8%, p<0.05 compared to background control mice).

PCSK9 knock-out mice had a blunted inflammatory cytokine response to LPS

In accordance with the inflammatory phenotype observations, PCSK9 knock-out mice also showed an attenuated pro-inflammatory cytokine response measured in plasma at 6 hours after LPS infusion (TABLE 2). More specifically, the PCSK9 knock-out mice had statistically lower median JE plasma concentrations of 25000 ± 11000 pg/ml compared to background control mice (37000 ± 9200 pg/ml) at 6 hours after LPS administration (P<0.05). MIP-2 was also reduced in PCSK9 knock-out mice with a median concentration of 54000 ± 13000 vs 63000 ± 6500 pg/ml in background controls (P<0.05). There was no difference in MFI between PCSK9 knock-out mice and background controls with respect to JE and MIP-2 in saline treated mice (TABLE 2).

**TABLE 2**

| Multiplex cytokine analysis of mouse plasma 6 hours following 20 mg/kg LPS. n=8 per group. Data are median concentration in pg/ml with standard deviation (P < 0.05 vs Background strain LPS) | | | | |
|---|---|---|---|---|
| ***Cytokine*** | ***Background Strain SL*** | ***PCSK9 KO SL*** | ***Background Strain LPS*** | ***PCSK9 KO LPS*** |
| TNFalpha | 1.7 ± .60 | 1.7 ± 3.0 | 224 ± 76 | 190 ± 34 |
| IL-6 | 8.1 ± 6.7 | 8.1 ± 108 | | |
| IL-10 | 1.2 ± 0 | 1.9 ± 5.9 | 520 ± 170 | 290 ± 100 |
| JE | 60 ± 30 | 30 ± 250 | 37000 ± 9200 | 25000 ± 11000* |
| MIP-2 | 4 ± 2 | 3 ± 6 | 63000 ± 6500 | 54000 ± 13000* |
| KC | 410 ± 600 | 280 ± 1000 | | |

### EXAMPLE 2: PCSK9 genotype is associated with mortality in humans

Using the four selected haplotype tag SNPs, it was found that PCSK9 genotype in humans was significantly associated with mortality (TABLE 3). No consistent significant differences at baseline characteristics in these two cohorts were identified that could potentially confound this result (TABLE 4). In particular, the minor G allele of rs644000 A/G was highly associated with decreased mortality over 28 days in both the SPH cohort (p<0.0045) and in the VASST cohort (0.0044) (TABLE 5 and FIGURE 2). In accord with this observation, it was also found that the G allele of rs644000 was associated with decreased dysfunction of cardiovascular, respiratory, renal and hepatic organ systems and trends to more neurologic and hematologic dysfunction (as expressed by decreased numbers of days alive and free of organ dysfunction in TABLE 6). There was also a significantly increased need for vasopressor use, mechanical ventilation, and renal replacement therapy (TABLE 6). The results in TABLE 6 show that respiratory failure resulted in subjects with severe infection and correlated with rs644000 genotype.

**TABLE 3**

| Associations of haplotype tag SNPs with 28-day mortality in SPH. | | | | | |
|---|---|---|---|---|---|
| **Gene** | **rs #** | **Major (minor allele)** | **Minor allele frequency (HapMap)** | **HWE P value** | **Raw P value (Corrected *P* value)** |
| PCSK9 | rs2479408 | C(G) | 0.178(0.186) | 0.38 | 0.043(0.17) |
| | rs2479409 | A(G) | 0.441(0.350) | 9.8 x 10⁻⁹ | 0.18 |
| | rs572512 | C(T) | 0.495(0.381) | 5.6 x 10⁻⁷ | 0.55 |
| | rs644000 | A(G) | 0.301(0.371) | 0.21 | 0.0070(0.028) |

**TABLE 4**

| Baseline characteristics of patients in the SPH and VASST cohorts by PCSK9 rs644000 genotype. | | | | | | | | |
|---|---|---|---|---|---|---|---|---|
| PCSK9 rs644000 genotype | SPH Cohort | | | | VASST Cohort | | | |
| | GG | GA | AA | | GG | GA | AA | |
| | (n=60) | (n=234) | (n=295) | *P* | (n=77) | (n=273) | (n=266) | *P* |
| Age (yrs.) | 63(51-72) | 62(49-72) | 62(46-73) | 0.89 | 65(52-73) | 63(49-73) | 63(50-73) | 0.55 |
| Gender -% male | 73.3 | 62.4 | 61.4 | 0.21 | 63.6 | 57.9 | 59.4 | 0.66 |
| Caucasian -n (%) APACHE II | 48(80.0) | 194(82.9) | 211(71.5) | 0.80 | 74(96.1) | 236(86.4) | 207(77.8) | 0.66 |
| Surgical -% | 24(18-29) 25.0 | 26(21-31) 29.1 | 27(20-33) 31.5 | 0.16 0.67 | 26(20-31) | 26(21-32) | 27(23-32) | 0.19 |
| Pre-existing conditions -n (%) | | | | | | | | |
| Chronic heart failure | 4(6.7) | 20(8.5) | 13(4.4) | 0.15 | 8(10.4) | 14(5.1) | 25(9.4) | 0.11 |
| Chronic pulmonary disease | 10(16.7) | 41(17.5) | 49(16.6) | 0.96 | 11(14.3) | 45(16.5) | 52(19.5) | 0.47 |
| Chronic liver disease | 6(10.0) | 18(7.7) | 34(11.5) | 0.34 | 10(13.0) | 31(11.4) | 27(10.2) | 0.76 |
| Chronic renal failure | 5(8.3) | 12(5.1) | 22(7.5) | 0.48 | 8(10.4) | 23(8.4) | 37(13.9) | 0.13 |
| Chronic corticosteroid use | 5(8.3) | 13(5.6) | 20(6.8) | 0.70 | 13(16.9) | 49(17.9) | 67(25.2) | 0.077 |
| Cardiovascular variables -Day 1 | | | | | | | | |
| Heart rate -bpm | 106(95-120) | 110(95-130) | 115(96-135) | 0.061 | 120(108-134) | 125(112-140) | 130(110-140) | 0.11 |
| Mean arterial pressure - mmHg | 54(50-58) | 55(50-59) | 55(49-59) | 0.45 | 56(50-62) | 56(50-62) | 55(50-60) | 0.40 |
| Central venous pressure - mmHg | 11(6-13) | 11(7-15) | 12(8-15) | 0.22 | 14(12-18) | 14(11-18) | 14(11-18) | 0.78 |
| Norepinephrine -µg/min | 10(6-23) | 15(7-25) | 15(8-29) | 0.59 | 16(10-27) | 14(8-25) | 16(10-29) | 0.074 |
| Dobutamine -µg/kg/min | 5(3-10) | 7(5-10) | 8(5-12) | 0.40 | 5(4-7) | 4(3-8) | 4(2-6) | 0.61 |
| Laboratory variables- Dayl | | | | | | | | |
| White blood cell count - 10³/mm³ | 14.6(10.2-21.6) | 15.6(10.4-21.3) | 14.1(9.3-19.0) | 0.22 | 14.5(9.2-20.6) | 13.9(7.5-21.1) | 12.5(7.0-19.8) | 0.38 |
| Platelet count -10³/mm³ | 206(85-300) | 162(95-243) | 161(90-240) | 0.19 | 145(88-235) | 157(91-242) | 147(60-259) | 0.63 |
| PaO₂/F_{I}O₂ -torr | 160(91-216) | 142(93-220) | 145(89-214) | 0.91 | 200(155-255) | 188(131-253) | 188(132-263) | 0.59 |
| Blood creatinine -µmol/L | 142(80-271) | 145(83-283) | 150(90-272) | 0.69 | 147(87-245) | 150(90-240) | 154(97-272) | 0.23 |
| Blood lactate -mmol/L | 2.0(1.4-5.9) | 2.3(1.4-4.4) | 2.3(1.4-5.1) | 0.89 | 2.1(1.4-4.4) | 2.3(1.4-4.1) | 2.3(1.4-4.6) | 0.66 |

**TABLE 5**

| Logistic regression of rs644000 genotype with 28-day mortality in SPH and VASST cohorts. | | | | | | | | |
|---|---|---|---|---|---|---|---|---|
| | | SPH Cohort Odds Ratio (95% Confidence interval) | | *P* | VASST Cohort Odds Ratio (95% Confidence interval) | | *P* | |
| Age -per year | | 1.028 (1.016-1.038) | | 1.1x10⁻⁶ | 1.019 (1.0080-1.030) | | 6.2x10⁻⁴ | |
| Female | | 0.87 (0.61-1.23) | | 0.43 | 0.93 (0.66-1.32) | | 0.71 | |
| Caucasian | | 0.87 (0.58-1.30) | | 0.49 | 0.81 (0.51-1.29) | | 0.37 | |
| Surgical | | 0.78 (0.54-1.14) | | 0.20 | 0.76 (0.50-1.17) | | 0.21 | |
| PCSK9 rs644000 A allele | | 1.46 (1.12-1.88) | | 0.0045 | 1.46 (1.12-1.89) | | 0.0044 | |
| | | | | | | | | |

| **TABLE 6** | | | | | | | | |
|---|---|---|---|---|---|---|---|---|
| Organ dysfunction (DAF) in SPH and VASST cohorts by rs644000 genotype. | | | | | | | | |
| | **SPH Cohort** | | | | **VASST Cohort** | | | |
| **PCSK9 rs644000 genotype** | **GG** | **GA** | **AA** | | **GG** | **GA** | **AA** | |
| | **(n=60)** | **(n=234)** | **(n=295)** | ***P*** | **(n=77)** | **(n=273)** | **(n=266)** | ***P*** |
| **Days alive and free Organ dysfunction** | | | | | | | | |
| **Cardiovascular** | 18(5-25) | 15(1-24) | 8(0-23) | 0.0086 | 21(2-24) | 19(0-24) | 14(0-23) | 0.028 |
| **Respiratory** | 18(1-25) | 10(0-24) | 4(0-22) | 0.0091 | 10(1-15) | 3(0-16) | 2(0-15) | 0.11 |
| **Renal** | 22(3-28) | 17(2-28) | 11(1-27) | 0.062 | 26(12-28) | 23(6-28) | 18(2-28) | 0.0028 |
| **Hematologic** | 27(6-28) | 24(6-28) | 20(3-28) | 0.068 | 27(10-28) | 26(9-28) | 22(2-28) | 0.0050 |
| **Hepatic** | 28(6-28) | 24(4-28) | 17(2-28) | 0.041 | 28(8-28) | 27(7-28) | 22(4-28) | 0.026 |
| **Neurologic** | 26(8-28) | 24(5-28) | 20(3-27) | 0.084 | 19(5-26) | 15(0-24) | 15(0-23) | 0.029 |
| **Artificial organ support** | | | | | | | | |
| **Vasopressor** | 24(9-27) | 21(2-26) | 17(1-26) | 0.019 | 22(2-25) | 20(0-24) | 16(0-24) | 0.028 |
| **Ventilator** | 17(0-23) | 8(0-22) | 2(0-21) | 0.019 | 13(0-22) | 8(0-20) | 8(0-20) | 0.26 |
| **Renal replacement therapy** | 28(4-28) | 23(3-28) | 12(1-28) | 0.0082 | 28(11-28) | 27(9-28) | 20(3-28) | 0.0074 |

### Human PCSK9 Loss-Of-Function and Gain-Of-Function variants

The human PCSK9 gene has been highly characterized and several relatively common missense variants³¹ (Minor Allele Frequency [MAF] ≥0.5%) and many rare missense and nonsense variants³² have been identified that are associated with decreased LDL levels³²⁻³⁴, as an indicator of Loss-Of-Function (LOF) of PCSK9 (FIGURE 3). The relationship between LDL levels and genotype of PCSK9 has also been identified using an unbiased GWAS approach³⁵. One fairly common missense variant³⁶ and many rare missense variants^{31,37} have been found to be associated with increased LDL levels and, based on this, are considered Gain-Of-Function variants (GOF) (FIGURE 3). Accordingly, we genotyped the relatively common PCSK9 LOF variants (MAP≥0.5%, rs11591147 R46L, rs11583680 A53V, rs562556 V474I) and the relatively common PCSK9 GOF variant (rs505151 G670E) in the VASST cohort.

It was found that the LOF alleles of rs11591147, rs11583680, and rs562556 all preferentially segregated with the minor G allele of our tag SNP, rs644000 (FIGURE 3). Out of 1324 total observed haplotypes a LOF allele was observed 442 times within 309 haplotypes. 83.5% of these LOF alleles were contained within haplotypes that also contained the rs644000 minor G allele. Only 15.5% of the LOF alleles were contained within rs644000 major A allele haplotypes. Thus, the minor allele of rs644000 is a marker of the most common PCSK9 LOF genetic variants. Conversely, the relatively common GOF variant preferentially segregated with the major A allele of rs644000 (FIGURE 3). 95.2% of these GOF alleles were contained within haplotypes that also contained the rs644000 major A allele. Only 4.8% of the GOF alleles were contained within the rs644000 minor G allele haplotypes. Thus, overall, the minor allele of rs644000 is preferentially associated with LOF alleles and the major allele of rs644000 is preferentially associated with GOF alleles of know relatively common genetic variants of PCSK9. To understand whether PCSK9 rs644000 was highly associated with patient outcome because it or a SNP in high linkage disequilibrium (LD) with it was a functional SNP or, conversely, whether the associated LOF and GOF variants were the likely functional SNPs, the LOF and GOF variants were first separately examined.

It was found that patients in VASST having at least one PCSK9 LOF allele had decreased mortality over 28 days (29.1% 28-day mortality, FIGURE 4A) compared to patients without a LOF allele (39.6% 28-day mortality, FIGURE 4A) (p=0.0037 by log-rank test). Using logistic regression to identify and adjust for potentially important covariates, it was found that having at least one PCSK9 LOF allele remained statistically significant (P<0.009) (TABLE 7).

**TABLE 7**

| Logistic regression testing for LOF and GOF effects on 28-day mortality in VASST. | | | | |
|---|---|---|---|---|
| LOF patients had at least one LOF allele. GOF patients had at least one GOF allele | | | | |
| | Loss-Of-Function Odds Ratio (95% Confidence interval) | *P* | Gain-Of-Function Odds Ratio (95% Confidence interval) | *P* |
| Age -per year | 1.017 (1.007-1.029) | 0.001 | 1.007 (0.997-1.027) | 0.11 |
| Female | 0.99 (0.71-1.40) | 0.97 | 0.81 (0.50-1.29) | 0.37 |
| Caucasian | 0.80 (0.51-1.26) | 0.33 | 0.91 (0.46-1.77) | 0.77 |
| Surgical | 0.75 (0.50-1.14) | 0.18 | 0.64 (0.51-1.52) | 0.63 |
| Effect of genetic variant | 0.64 (0.46-0.89) | 0.009 | 1.92 (1.06-3.48) | 0.031 |

The number of haplotypes containing a GOF variant (n=59+3 rare=62 in FIGURE 3) was less than the number containing at least one of the three LOF variants (total n = 108+136+54+6+5 rare=309 in FIGURE 3). Therefore, there was no expectation that a statistically significant effect of the GOF variant would be found, but there was interest in determining if GOF effects were directionally opposite LOF effects. Indeed, it was found that patients in VASST having the GOF variant had a directionally opposite effect to LOF variants; having increased mortality over 28 days (43.9% 28-day mortality, FIGURE 4B) compared to patients with a LOF variant (28.3% 28-day mortality, FIGURE 4B) (p=0.011 by log-rank test). Logistic regression, adjusting for potentially important covariates, similarly identified increased mortality in patients having a PCSK9 GOF variant (p=0.031) (TABLE 7).

To further distinguish between a primary effect of rs644000 (or a SNP in LD) versus a primary effect of LOF variants, it was reasoned that if the primary effect was due to rs644000, and not due to LOF variants, then an analysis that selected only heterozygotes for rs644000 (thereby keeping the rs644000 genetic background constant) would eliminate the LOF effect. However, it was found that LOF variants retained a statistically significant decrease in mortality effect even in these selected patients (FIGURE 5). These rs644000 heterozygous patients in VASST having at least one PCSK9 LOF allele had decreased mortality (28.9% 28-day mortality) compared to patients without a LOF allele (37.8% 28-day mortality) (p=0.029 by log-rank test). Thus, it appeared that the effect on mortality was most likely conferred by LOF variants (and GOF variants) rather than by a primary effect of rs644000. Most likely, rs644000 was simply a marker of preponderance of LOP variants associated with the minor G allele of rs644000 and GOF variant almost exclusively associated with the major A allele of rs644000.

PCSK9 knock-out mice when compared to genetic background controls, were protected against the adverse effects of LPS administration, which is an important pre-clinical model of the systemic inflammatory response relevant to sepsis and septic shock. The PCSK9 knock-out mice showed beneficial amelioration of the adverse effect of LPS on activity and body temperature, as well as on adverse LPS-induced cardiovascular phenotypes of hypotension and decreased left ventricular ejection fraction. PCSK9 knock-out mice also showed a decrease in the plasma inflammatory cytokine response in response to LPS. Knocking out PCSK9 proved to be protective of the important and common adverse effects of LPS. Furthermore, to determine whether the impact of PCSK9 was relevant in human subjects a tag SNP approach was used, whereby well characterized PCSK9 Loss-Of-Function (LOF) and Gain-Of-Function (GOF) genetic variants were utilized similar to the PCSK9 knockout mice. It is shown herein that genotype of the PCSK9 tag SNP, rs644000, was highly associated with 28-day mortality in the SPH septic shock cohort and this result replicated in the VASST septic shock cohort. It was also shown herein that LOF missense variants were associated with decreased 28-day mortality while GOF missense variants were associated with increased 28-day mortality. All three relatively common LOF variants (MAF≥0.5%) were predominately found within haplotypes tagged by the minor allele of rs644000 and the relatively common GOF variant was almost exclusively found within haplotypes tagged by the major allele of rs644000, likely accounting for the strong association of the minor G allele of rs644000 with decreased 28-day mortality. Furthermore, it was found that plasma concentrations of inflammatory cytokines and chemokines were elevated in patients having GOF variants compared to those with LOF variants. These human septic shock results align well with the mouse model of systemic inflammation indicating that decreased PCSK9 activity results in a decreased inflammatory cytokine response and increased survival. These results support the conclusion that reduction of activity of PCSK9 reduces the inflammatory response and improves physiologic outcome in mice following LPS administration and also increases survival and reduces renal failure or renal dysfunction in human subjects with severe sepsis and septic shock.

### LOF and GOF Mutations in PCSK9

Many mutations in the PCSK9 gene have already been well documented³¹ and functional effects characterized^{40,44}. LOF mutations result in reduced sequestration of the LDL receptor and, as a result, decreased plasma LDL cholesterol concentrations. Presumably LOF mutations would similarly impact other receptors regulated by PCSK9 including the very low-density lipoprotein receptor, ApoER2, and CD81. Three LOF variants have been found to be relatively common (MAF>0.5%). The missense variant rs11591147 results in an R46L substitution, which is consistently associated with low LDL cholesterol levels and, hence is a LOF variant^{31,32,45}. This variant results in improved patient outcomes in atherosclerotic disease⁴⁶. Similarly, rs11583680 A53V is common and reported to be a LOF variant³¹. The missense variant rs562556 results in a V474I substitution, which results in decreased total cholesterol and LDL cholesterol⁴⁷. Conversely, GOF variants lead to decreased numbers of LDL receptors at the cell surface of hepatocytes and, as a result, increased plasma LDL cholesterol levels with a concomitant increase in atherosclerosis and cardiovascular disease⁴⁸. Some genetic variants in PCSK9 may result in LOF or GOF by affecting the affinity with which PCSK9 binds the LDL receptor, with some variants displaying a many-fold increase in affinity over wild type PCSK9⁴⁰. Other genetic variants may affect the degradation of PCSK9 protein, either increasing or decreasing the half-life of PCSK9⁴⁹.

### PCSK9 in Inflammation and Infection

PCSK9 results in up-regulation of genes involved in sterol biosynthesis and down-regulation of stress-response genes and specific inflammation pathways⁵⁰. PCSK9 has pro-apoptotic effects⁵¹. PCSK9 concentrations are increased in patients who have peri-odontal infections; PCSK9 concentrations in periodontitis patients were significantly higher than healthy controls in one study⁵². Labonte and colleagues found an antiviral effect of circulating liver-derived PCSK9 on HCV in cells and showed that PCSK9 down-regulates the level of mouse liver CD81 expression *in vivo.* Cells expressing PCSK9 were shown to be resistant to HCV infection. Also, addition of purified soluble PCSK9 to cell culture supernatant impeded HCV infection in a dose-dependent manner.

The results from human septic shock align in many ways with the mouse model of systemic inflammation. Therefore, it is concluded that decreased PCSK9 activity results in amelioration of adverse consequences of systemic inflammation including adverse cardiovascular effects (hypotension, decreased left ventricular ejection fraction in mice; cardiovascular organ dysfunction and need for vasoactive agents in humans), adverse global phenotypes (activity and body temperature in mice; multiple organ dysfunction in humans), amelioration of the inflammatory cytokine response (similar pattern in mice and humans), and reduction in mortality (surrogate mortality endpoints in mice, 28-day mortality in humans). There are a number of PCSK9 inhibitors in development for treatment of lipid disorders, but none are focused on sepsis or septic shock or other related inflammatory conditions. A reduction in PCSK9 activity using PCSK9 inhibition could improve outcomes of human sepsis, septic shock, and many other inflammatory responses to infection, or reduce/prevent treating or preventing renal failure; renal dysfunction; respiratory failure; respiratory dysfunction; or acute lung injury.

Numeric ranges are inclusive of the numbers defining the range. The word "comprising" is used herein as an open-ended term, substantially equivalent to the phrase "including, but not limited to", and the word "comprises" has a corresponding meaning. As used herein, the singular forms "a", "an" and "the" include plural referents unless the context clearly dictates otherwise. Thus, for example, reference to "a thing" includes more than one such thing.

### REFERENCES

1. Novack V, Macfadyen J, Malhotra A, et al. The effect of rosuvastatin on incident pneumonia: Results from the jupiter trial. CMAJ. 2012;184:E367-372.
2. Rothberg MB, Bigelow C, Pekow PS, Lindenauer PK. Association between statins given in hospital and mortality in pneumonia patients. J Gen Intern Med. 2012;27:280-286.
3. Grion CM, Cardoso LT, Perazolo TF, et al. Lipoproteins and cetp levels as risk factors for severe sepsis in hospitalized patients. Eur J Clin Invest. 2010;40:330-338.
4. Chalmers JD, Short PM, Mandal P, Akram AR, Hill AT. Statins in community acquired pneumonia: Evidence from experimental and clinical studies. Respir Med. 2010;104:1081-1091.
5. Kruger PS, Harward ML, Jones MA, et al. Continuation of statin therapy in patients with presumed infection: A randomized controlled trial. Am JRespir Crit Care Med. 2011;183:774-781.
6. Barter PJ, Caulfield M, Eriksson M, et al. Effects of torcetrapib in patients at high risk for coronary events. NEngl JMed. 2007;357:2109-2122.
7. Harris HW, Grunfeld C, Feingold KR, et al. Chylomicrons alter the fate of endotoxin, decreasing tumor necrosis factor release and preventing death. J Clin Invest. 1993;91:1028-1034.
8. Read TE, Harris HW, Grunfeld C, et al. The protective effect of serum lipoproteins against bacterial lipopolysaccharide. Eur Heart J. 1993;14 Suppl K:125-129.
9. Read TE, Grunfeld C, Kumwenda ZL, et al. Triglyceride-rich lipoproteins prevent septic death in rats. J Exp Med. 1995;182:267-272.
10. Harris HW, Rockey DC, Chau P. Chylomicrons alter the hepatic distribution and cellular response to endotoxin in rats. Hepatology. 1998;27:1341-1348.
11. de Lima-Salgado TM, Cruz LM, Souza HP. Lipid-activated nuclear receptors and sepsis. Endocr Metab Immune Disord Drug Targets. 2010;10:258-265.
12. Thompson PA, Berbee JF, Rensen PC, Kitchens RL. Apolipoprotein a-ii augments monocyte responses to lps by suppressing the inhibitory activity of lps-binding protein. Innate Immun. 2008;14:365-374.
13. Abe M, Maruyama N, Okada K, Matsumoto S, Matsumoto K, Soma M. Effects of lipid-lowering therapy with rosuvastatin on kidney function and oxidative stress in patients with diabetic nephropathy. JAtheroscler Thromb. 2011;18:1018-1028.
14. Fraunberger P, Grone E, Grone HJ, Walli AK. Simvastatin reduces endotoxin-induced nuclear factor kappab activation and mortality in guinea pigs despite lowering circulating low-density lipoprotein cholesterol. Shock. 2009;32:159-163.
15. McGown CC, Brown NJ, Hellewell PG, Reilly CS, Brookes ZL. Beneficial microvascular and anti-inflammatory effects of pravastatin during sepsis involve nitric oxide synthase iii. Br J Anaesth. 2010;104:183-190.
16. Winkler F, Angele B, Pfister HW, Koedel U. Simvastatin attenuates leukocyte recruitment in experimental bacterial meningitis. Int Immunopharmacol. 2009;9:371-374.
17. Yang W, Yamada M, Tamura Y, et al. Farnesyltransferase inhibitor fti-277 reduces mortality of septic mice along with improved bacterial clearance. J Pharmacol Exp Ther. 2011;339:832-841.
18. Netea MG, Demacker PN, Kullberg BJ, et al. Low-density lipoprotein receptor-deficient mice are protected against lethal endotoxemia and severe gram-negative infections. J Clin Invest. 1996;97:1366-1372.
19. Lanza-Jacoby S, Miller S, Jacob S, et al. Hyperlipoproteinemic low-density lipoprotein receptor-deficient mice are more susceptible to sepsis than corresponding wild-type mice. J Endotoxin Res. 2003;9:341-347.
20. Rosch JW, Boyd AR, Hinojosa E, et al. Statins protect against fulminant pneumococcal infection and cytolysin toxicity in a mouse model of sickle cell disease. J Clin Invest. 2010;120:627-635.
21. Zhang Z, Datta G, Zhang Y, et al. Apolipoprotein a-i mimetic peptide treatment inhibits inflammatory responses and improves survival in septic rats. Am JPhysiol Heart Circ Physiol. 2009;297:H866-873.
22. Steinberg D, Witztum JL. Inhibition of pcsk9: A powerful weapon for achieving ideal ldl cholesterol levels. Proc Natl Acad Sci U S A. 2009;106:9546-9547.
23. Rashid S, Curtis DE, Garuti R, Anderson NN, Bashmakov Y, Ho YK, Hammer RE, Moon YA, Horton JD. Decreased plasma cholesterol and hypersensitivity to statins in mice lacking pcsk9. Proc Natl Acad Sci USA. 2005;102:5374-5379.
24. Boyd JH, Mathur S, Wang Y, Bateman RM, Walley KR. Toll-like receptor stimulation in cardiomyoctes decreases contractility and initiates an nf-kappab dependent inflammatory response. Cardiovasc Res. 2006;72:384-393.
25. American college of chest physicians/society of critical care medicine consensus conference: Definitions for sepsis and organ failure and guidelines for the use of innovative therapies in sepsis. Crit Care Med. 1992;20:864-874.
26. Walley KR, Russell JA. Protein c -1641 aa is associated with decreased survival and more organ dysfunction in severe sepsis. Crit Care Med. 2007;35:12-17.
27. Russell JA, Walley KR, Singer J, et al. Vasopressin versus norepinephrine infusion in patients with septic shock. N Engl J Med. 2008;358:877-887
28. Stephens M, Donnelly P. A comparison of bayesian methods for haplotype reconstruction from population genotype data. Am J Hum Genet. 2003;73:1162-1169.
29. Manocha S, Russell JA, Sutherland AM, Wattanathum A, Walley KR. Fibrinogen-beta gene haplotype is associated with mortality in sepsis. J Infect. 2007;54:572-577.
30. Boyd JH, Kan B, Roberts H, Wang Y, Walley KR. S100a8 and s100a9 mediate endotoxin-induced cardiomyocyte dysfunction via the receptor for advanced glycation end products. Circ Res. 2008;102:1239-1246.
31. Kotowski IK, Pertsemlidis A, Luke A, et al. A spectrum of pcsk9 alleles contributes to plasma levels of low-density lipoprotein cholesterol. Am J Hum Genet. 2006;78:410-422.
32. Cohen JC, Boerwinkle E, Mosley TH, Jr., Hobbs HH. Sequence variations in pcsk9, low ldl, and protection against coronary heart disease. N Engl J Med. 2006;354:1264-1272.
33. Talmud PJ, Drenos F, Shah S, et al. Gene-centric association signals for lipids and apolipoproteins identified via the humancvd beadchip. Am J Hum Genet. 2009;85:628-642.
34. Musunuru K, Lettre G, Young T, et al. Candidate gene association resource (care): Design, methods, and proof of concept. Circ Cardiovasc Genet. 2010;3:267-275.
35. Kathiresan S, Voight BF, Purcell S, et al. Genome-wide association of early-onset myocardial infarction with single nucleotide polymorphisms and copy number variants. Nat Genet. 2009;41:334-341.
36. Abifadel M, Varret M, Rabes JP, et al. Mutations in pcsk9 cause autosomal dominant hypercholesterolemia. Nat Genet. 2003;34:154-156.
37. Blesa S, Vernia S, Garcia-Garcia AB, et al. A new pcsk9 gene promoter variant affects gene expression and causes autosomal dominant hypercholesterolemia. J Clin Endocrinol Metab. 2008;93:3577-3583.
38. Naureckiene S, Ma L, Sreekumar K, et al. Functional characterization of narc 1, a novel proteinase related to proteinase k. Arch Biochem Biophys. 2003;420:55-67.
39. Benjannet S, Rhainds D, Essalmani R, et al. Narc-l/pcsk9 and its natural mutants: Zymogen cleavage and effects on the low density lipoprotein (ldl) receptor and ldl cholesterol. JBiol Chem. 2004;279:48865-48875.
40. Cunningham D, Danley DE, Geoghegan KF, et al. Structural and biophysical studies of pcsk9 and its mutants linked to familial hypercholesterolemia. Nat Struct Mol Biol. 2007;14:413-419.
41. Qian YW, Schmidt RJ, Zhang Y, et al. Secreted pcsk9 downregulates low density lipoprotein receptor through receptor-mediated endocytosis. J Lipid Res. 2007;48:1488-1498.
42. Zhang DW, Lagace TA, Garuti R, et al. Binding of proprotein convertase subtilisin/kexin type 9 to epidermal growth factor-like repeat a of low density lipoprotein receptor decreases receptor recycling and increases degradation. J Biol Chem. 2007;282:18602-18612.
43. Stein EA, Mellis S, Yancopoulos GD, et al. Effect of a monoclonal antibody to pcsk9 on ldl cholesterol. N Engl J Med. 2012;366:1108-1118.
44. Fisher TS, Lo Surdo P, Pandit S, et al. Effects of ph and low density lipoprotein (ldl) on pcsk9-dependent ldl receptor regulation. JBiol Chem. 2007;282:20502-20512.
45. Scartezini M, Hubbart C, Whittall RA, et al. The pcsk9 gene r461 variant is associated with lower plasma lipid levels and cardiovascular risk in healthy u.K. Men. Clin Sci (Lond). 2007; 113:435-441.
46. Kathiresan S. A pcsk9 missense variant associated with a reduced risk of early-onset myocardial infarction. N Engl J Med. 2008;358:2299-2300.
47. Shioji K, Mannami T, Kokubo Y, et al. Genetic variants in pcsk9 affect the cholesterol level in japanese. J Hum Genet. 2004;49:109-114.
48. Horton JD, Cohen JC, Hobbs HH. Molecular biology of pcsk9: Its role in ldl metabolism. Trends Biochem Sci. 2007;32:71-77.
49. Benjannet S, Rhainds D, Hamelin J, Nassoury N, Seidah NG. The proprotein convertase (pc) pcsk9 is inactivated by furin and/or pc5/6a: Functional consequences of natural mutations and post-translational modifications. JBiol Chem. 2006;281:30561-30572
50. Ranheim T, Mattingsdal M, Lindvall JM, et al. Genome-wide expression analysis of cells expressing gain of function mutant d374y-pcsk9. J Cell Physiol. 2008;217:459-467.
51. Bingham B, Shen R, Kotnis S, et al. Proapoptotic effects of narc 1 (= pcsk9), the gene encoding a novel serine proteinase. Cytometry A. 2006;69:1123-1131.
52. Miyazawa H, Honda T, Miyauchi S, et al. Increased serum pcsk9 concentrations are associated with periodontal infection but do not correlate with ldl cholesterol concentration. Clin Chim Acta. 2012;413:154-159.
53. Lambert G, Sjouke B, Choque B, Kastelein JJ, Hovingh GK. The PCSK9 decade. J Lipid Res. 2012;53(12):2515-24.

## Claims

1. A proprotein convertase subtilisin kexin 9 (PCSK9) inhibitor for use in the treatment of sepsis or septic shock, wherein the PCSK9 inhibitor is an antibody or antigen-binding fragment thereof.

2. The PCSK9 inhibitor for use according to claim 1, wherein the PCSK9 inhibitor is a monoclonal antibody or antigen-binding fragment thereof.

3. The PCSK9 inhibitor for use according to claim 1 or 2, wherein the PCSK9 inhibitor is: AMG145; 1D05-IgG2; SAR236553/REGN727 (Alirocumab); RN-316; LGT209; or RG7652.

4. The PCSK9 inhibitor for use according to any one of claims 1 to 3, wherein the subject is a human.

5. A pharmaceutical composition for use in the treatment of sepsis or septic shock, comprising a PCSK9 inhibitor, wherein the PCSK9 inhibitor is an antibody or antigen-binding fragment thereof.

6. The pharmaceutical composition for use according to claim 5, wherein the PCSK9 inhibitor is a monoclonal antibody or antigen-binding fragment thereof.

7. The pharmaceutical composition for use according to claim 6, wherein the PCSK9 inhibitor is: AMG145; 1D05-IgG2; SAR236553/REGN727 (Alirocumab); RN-316; LGT209; or RG7652.

## Patentansprüche

1. Proprotein-Convertase-Subtilisin-Kexin-9-(PCSK9)-Inhibitor zur Verwendung bei der Behandlung von Sepsis oder septischem Schock, wobei der PCSK9-Inhibitor ein Antikörper oder ein Antigen-bindendes Fragment davon ist.

2. PCSK9-Inhibitor zur Verwendung nach Anspruch 1, wobei der PCSK9-Inhibitor ein monoklonaler Antikörper oder ein Antigen-bindendes Fragment davon ist.

3. PCSK9-Inhibitor zur Verwendung nach Anspruch 1 oder 2, wobei der PCSK9-Inhibitor Folgendes ist: AMG145; 1D05-IgG2; SAR236553/REGN727 (Alirocumab); RN-316; LGT209 oder RG7652.

4. PCSK9-Inhibitor zur Verwendung nach einem der Ansprüche 1 bis 3, wobei das Subjekt ein Mensch ist.

5. Pharmazeutische Zusammensetzung zur Verwendung bei der Behandlung von Sepsis oder septischem Schock, die einen PCSK9-Inhibitor umfasst, wobei der PCSK9-Inhibitor ein Antikörper oder ein Antigen-bindendes Fragment davon ist.

6. Pharmazeutische Zusammensetzung zur Verwendung nach Anspruch 5, wobei der PCSK9-Inhibitor ein monoklonaler Antikörper oder ein Antigen-bindendes Fragment davon ist.

7. Pharmazeutische Zusammensetzung zur Verwendung nach Anspruch 6, wobei der PCSK9-Inhibitor Folgendes ist: AMG145; 1D05-IgG2; SAR236553/REGN727 (Alirocumab); RN-316; LGT209 oder RG7652.

## Revendications

1. Inhibiteur de proprotéine convertase subtilisine/kexine 9 (PCSK9) destiné à être utilisé dans le traitement de la sepsie ou du choc septique, lequel inhibiteur de PCSK9 est un anticorps ou un fragment liant l'antigène de celui-ci.

2. Inhibiteur de PCSK9 destiné à être utilisé selon la revendication 1, lequel inhibiteur de PCSK9 est un anticorps monoclonal ou un fragment liant l'antigène de celui-ci.

3. Inhibiteur de PCSK9 destiné à être utilisé selon la revendication 1 ou la revendication 2, lequel inhibiteur de PCSK9 est AMG145, ID05-IgG2, SAR236553/REGN727 (Alirocumab), RN-316, LGT209 ou RG7652.

4. Inhibiteur de PCSK9 destiné à être utilisé selon l'une quelconque des revendications 1 à 3, le sujet étant un humain.

5. Composition pharmaceutique destinée à être utilisée dans le traitement de la sepsie ou du choc septique, comprenant un inhibiteur de PCSK9, lequel inhibiteur de PCSK9 est un anticorps ou un fragment liant l'antigène de celui-ci.

6. Composition pharmaceutique destinée à être utilisée selon la revendication 5, l'inhibiteur de PCSK9 étant un anticorps monoclonal ou un fragment liant l'antigène de celui-ci.

7. Composition pharmaceutique destinée à être utilisée selon la revendication 6, l'inhibiteur de PCSK9 étant AMG145, ID05-IgG2, SAR236553/REGN727 (Alirocumab), RN-316, LGT209 ou RG7652.
